# EUROPEAN PATENT APPLICATION

(11) **EP 0 774 249 A2**
(43) Date of publication of application: **21.05.1997**
(21) Application number: 96307502.3
(22) Date of filing: 15.10.1996
(51) Int. Cl.: A61K 7/48

(54) **Topical composition containing specific flavanones**

(30) Priority: 27.10.1995 US 550124
(71) Applicant: UNILEVER PLC, London EC4P 4BQ (GB); UNILEVER N.V., 3013 AL Rotterdam (NL)
(72) Inventor: Cho, Suk Hyung, Monroeville, Pennsylvania 15146 (US); Gottlieb, Keith, Houston, Texas 77079 (US)
(74) Representative: Rots, Maria Johanna Francisca

(57) **Abstract**

Cosmetic compositions containing combinations of flavanones:eriodictyol and/or taxifolin combined with taxifolin and/or hesperetin. Alternatively, a flavanone is combined with a short-chain lipid. The compositions enhance keratinocyte differentiation in skin, thus decreasing skin dryness and decreasing appearance of wrinkles.

## Description

### FIELD OF THE INVENTION

The invention relates to topical compositions containing specific flavanones and to application to skin for conditioning, moisturization and improved appearance.

### BACKGROUND OF THE INVENTION

The top layer of human skin or the epidermis is composed of many different cell types including keratinocytes, melanocytes and langerhans cells. Keratinocytes are the major cell types of the epidermis comprising 75-90% of the total number of cells in the human epidermis. Within the epidermis the keratinocytes reside in four distinct stages of differentiation.

The basal layer rests on the basal lamina separating the epidermis from the dermis. These cells are large columnar, rapidly proliferating cells. These basal cells migrate upward within the epidermis, initiated by the process of differentiation. The layer above the basal cells is the spinous layer.

The cells in the spinous layer initiate the production of proteins characteristic of the differentiated epidermis. The granular layer, lying above the spinous layer, is characterized by electron-dense granules. This layer is responsible for the synthesis of lipid molecules required for the formation of the water impermeable barrier of the skin.

The topmost layer of the skin, the stratum corneum, is formed from the granular layer by the destruction of cellular organelles. The cells in the stratum corneum, corneocytes, contain extensively cross-linked proteins, surrounded by a highly resistant cell envelope. The corneocytes are embedded in a layer of specific lipid structures and this structure provides the protective barrier for the skin. The outermost layer of corneocytes is peeled off from the skin during the normal process of desquamation. Differentiation of the epidermal keratinocytes is the driving force for the normal desquamation process to occur. Epidermal differentiation is important for providing the essential protective environment in order to prevent loss of water from the skin and to maintain youthful appearance.

One of the main features of epidermal differentiation is the formation of an extensively cross-linked protein-enriched cornified envelope. Cornified envelope formation is initiated in the differentiated upper layers of the epidermis by the cross-linking of specific protein substrates by a keratinocyte specific enzyme, transglutaminase-1 (TG-1). This enzyme is responsible for the glutamyl lysine cross-linking of these proteins into the insoluble cornified envelope. This process of differentiation also occurs in vitro in cultured keratinocytes (see Thacher et al., Cell, (1985), pp. 685-695) when the cells are induced to differentiate in response to prodifferentiating agents.

The present invention is based at least in part on the discovery that certain flavanones, namely hesperetin, naringenin, eriodictyol and taxifolin enhance keratinocyte differentiation.

Flavanones are generally recognized as safe molecules and are a sub-class of a family of flavonoids. The term "flavonoids" represents a very large group of compounds consisting of two aromatic rings joined by a three carbon unit, (e.g., C₆-C₃-C₆). The family of flavonoids includes monomeric flavonols, catechins, epicatechins (e.g., laurones), leucoanthocyanidins, proanthocyanidins, anthocyanidins, flavones, flavanones, chalcones, isoflavones, and neoflavones. Flavonoids are ubiquitous in plants. Although flavonoids do not seem to be of vital importance to the human diet, we consume flavonoids daily in fruits, vegetables, wine, cereals, drinks and food colorings.

Since the flavonoids are phenolic compounds, they act as potent antioxidants.

Cosmetic compositions employing flavonoids, in general, as antioxidants, are known. EP 0 595 694 A1 discloses a cosmetic composition containing an amino acid lauroyl methionate and flavonoids to inhibit free radical formation. Meadowsweet extract containing flavonoids as radical scavengers are disclosed in the abstract of EP 0 507 035 A. These documents do not disclose the sub-class of flavanones or any of the specific flavanones employed in the present invention, or the pro-differentiation effect of the flavanones on keratinocytes.

U.S. Patent No. 4,297,348 (Frazier) discloses a composition and method for the treatment of acne with naringin and naringenin. WO 94/23717 discloses the use of hesperetin for sebum control and acne treatment. As demonstrated in Example 1 below, naringin is not an effective material for promoting the differentiation of keratinocytes. Naringin is not a flavanone as defined in Formula I according to this invention. Although naringenin and hesperetin are both flavanones and are effective keratinocyte pro-differentiating agents, Frazier and WO '717 disclose these two specific flavanones for the treatment of acne, not for improving skin conditioning by promoting differentiation of keratinocytes. Furthermore, these documents do not mention other flavanones employed in the present invention and do not mention a synergistic effect of a combination of specific flavanones or the synergistic effect of a combination of specific flavanones with short-chain lipids on keratinocyte differentiation. Indeed, since both documents are directed at acne treatment, incorporation of lipids would defeat the purpose of the anti-acne compositions taught by both documents.

FR 2 687 572A discloses flavones for protection of skin from singlet oxygen. Other flavonoids are also disclosed (including naringenin, eriodictyol, and hesperetin). FR '572 does not mention the pro-differentiating effect on keratinocytes, does not mention a synergistic effect of a combination of specific flavanones or the synergistic effect of a combination of specific flavanones with short-chain lipids on keratinocyte differentiation.

### SUMMARY OF THE INVENTION

The present invention includes, in part, a topical composition containing:
(a) from 0.001% to 5.0wt% of a first flavanone selected from the group consisting of eriodictyol and/or taxifolin;
(b) from 0.001% to 5.0wt% of a second flavanone selected from the group consisting of naringenin and/or hesperetin; and
(c) a cosmetically acceptable vehicle for the flavanones.

In a preferred embodiment of the invention, the flavanones are combined with short-chain lipids to attain a synergystic increase in keratinocyte differentiation.

The invention further provides a topical composition containing:
(a) from 0.001 to 5.0 wt. % of a flavanone selected from the group consisting of eriodictyol, taxifolin, hesperetin, naringenin, and mixtures thereof;
(b) from 0.001 to 50 wt. % of a ceramide selected from the group consisting of a short-chain ceramide, pseudoceramide, and neoceramide; and
(c) a cosmetically acceptable vehicle for the flavanone and the ceramide.

The inventive compositions enhance keratinocyte differentiation, which in turn results in increased benefits to skin, such as improved skin conditioning, improved youthful appearance, a decrease in wrinkle appearance, moisturizing, and treatment of photodamaged skin.

Accordingly, the invention encompasses a cosmetic method of enhancing keratinocyte differentiation in skin, the method comprising applying to the skin a topical composition as described above.

The invention further encompasses a cosmetic method for treating the appearance of wrinkled, flaky, aged, or photodamaged skin comprising applying to the skin a topical composition as described above.

The present invention also includes use of the inventive composition in the manufacture of a medicament for use in treatment of dry skin, wrinkled or lined skin, or photodamaged skin.

### DESCRIPTION OF THE PREFERRED EMBODIMENT

Flavanones, namely hesperetin, naringenin, eriodictyol and taxifolin are the essential ingredients of the inventive composition, and have the following Formula **I**:

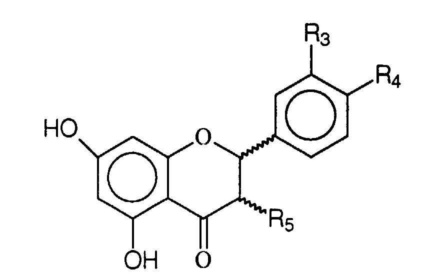

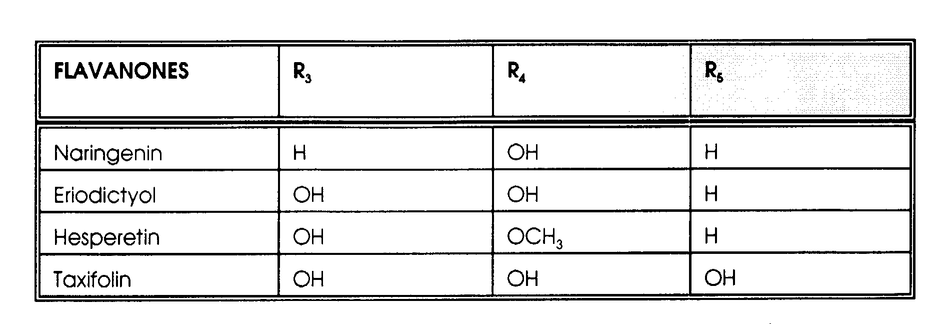

In one embodiment of the invention, the inventive compositions contain eriodictyol and/or taxifolin, in combination with a second flavanone, i.e., naringenin and/or hesperetin. The preferred compositions according to the invention further contain short-chain lipids, i.e., C₁-C₁₀ ceramides, pseudoceramides or neoceramides (described more fully below).

In the second embodiment of the invention, the inventive compositions contain naringenin, hesperetin, eriodictyol and/or taxifolin in combination with the short-chain lipid. Preferably they comprise naringenin and/or hesperetin as the flavanone.

Depending on the embodiment of the invention, the flavanone can be included in the inventive compositions in an amount ranging from 0.001 to 10 wt. %. In either case, the flavanone content is preferably from 0.005 to 5wt%, most preferably from 0.01 to 1%. In the first embodiment of the invention, the weight ratio of the first flavanone (i.e. eriodictyol and/or taxifolin) to the second flavanone (i.e. hesperetin and/or naringenin) is preferably in a range of from 1:5 to 5:1, more preferably from 1:3 to 3:1, most preferably from 1:2 to 2:1.

The short chain lipids are a preferred ingredient in the compositions according to the invention. The short-chain lipid is chosen from short-chain ceramides, short-chain pseudoceramides and short-chain neoceramides. By "short-chain" is meant that the group attached to the carbon atom of the nitrogen-linked C=O moiety contains 1-10 carbon atoms.

### Short-chain Ceramides:

Short-chain ceramides are preferably selected from ceramides having structure **(II)**: 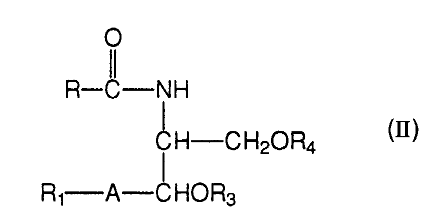 where A represents -CH₂- ; - CHOH- ; or -CH=CH-
R is a linear or branched saturated or unsaturated, aliphatic hydrocarbon group having from 1 to 10 carbon atoms which may contain a hydroxyl group:
R₁ represents a linear or branched, saturated or unsaturated, hydroxylated or non-hydroxylated aliphatic hydrocarbon group having from 8 to 28 carbon atoms or a phenyl group;
R₃ and R₄ individually represent H, a phosphate group or a sulfate group;

Further identification of ceramide structures may be found in U.S. Patent No. 4,950,688 (Bowser et al.).

Ceramides having the structure (II) are naturally occurring and can be isolated from a suitable plant source or from animal tissue such as pig skin or neural tissue. Ceramides can also be synthesized according to procedures described in one of the following references:
Shoyama, Y. et al., *Journal of Lipid Res.,* Vol. 19, (1978), pp. 250-258.
Hino, T. et al., *Journal of Chem. Soc. Parkin. Tran. J.* (1986), p. 1687.
Junana, R. et al., *Helv. Chem. Acta,* Vol. 69 (1986), p. 368.
Kiso, M. et al., *J. Carbohydrate Chem.,* Vol. 5, (1986), p. 93.
Kolke, K. et al., *Carbohyd. Res.,* Vol. 158, (1986), p. 113.
Schmidt, R. et al., *Tetrahedron. Let.,* (1986), pp. 481.

Ceramides may also be mixtures of different stereoisomers, (i.e., D-threo, L-threo, D-erythro and L-erythro). Most preferred, in order to attain the synergy with the flavonones are short chain ceramides wherein A is -CH₂, R₄ is hydrogen, R₃ is hydrogen, R₁ contains from 8 to 20 carbon atoms, and R contains from 1 to 7 carbon atoms.

### Short-Chain Pseudoceramides:

Short-chain pseudoceramides (i.e., synthetic ceramide-like structures) are preferably selected from pseudoceramides having the general structure **(III)**: 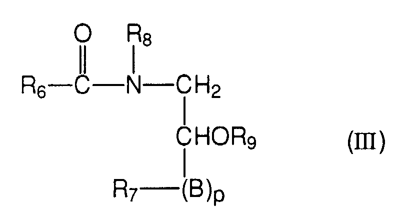 where B represents - OCH₂- or - CH₂CH(OH) or - CH₂;
R₆ represents a linear or branched, saturated or unsaturated, or hydroxylated aliphatic hydrocarbon group having from 1 to 10 carbon atoms;
R₇ represents a linear or branched, saturated or unsaturated or hydroxylated hydrocarbon group having from 8 to 28 carbon atoms or a phenyl group;
R₈ represents H, or a subgroup - (CH₂)_{c}R₁₀, or a subgroup having the structure (4), where c is an integer of from 1 to 6, R₁₀ is -OH or a phosphate group, or a sulfate group, or a sugar group; 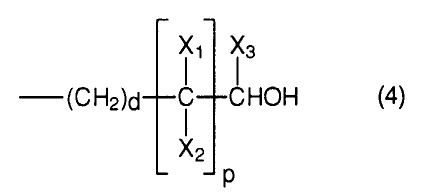 where X₁, X₂ and X₃ each individually represent H, a C₁₋₅ alkyl or a C ₁₋₅ hydroxyalkyl;
d is 0 or an integer of from 1 to 4; and
p is 0 or 1;
R₉ represents H, a phosphate group, a sulfate group or a sugar group.

Pseudoceramides may be synthesized according to the procedures described in U.S. Patent No. 4,778,823, or U.S. Patent No. 5,198,210, or U.S. Patent No. 5,206,020.

Preferably, in order to attain synergy and minimize cost, pseudoceramides are employed wherein R₈ is CH₂CH₂OH, R₉ is hydrogen, B is -OCH₂ or CH₂, and R₇ contains from 10 to 22 carbon atoms, R₆ = CH₃, CH₂CH₃, CH(OH)CH₃, or (CH₂)₄CH₃.

### Short-Chain Neoceramides:

Neoceramides, like pseudoceramides, are synthetic ceramide-like structures. Neoceramides, however, contain more localized polar groups than pseudoceramides. Neoceramides are preferably selected from neoceramides having the general structure **(IV)**: 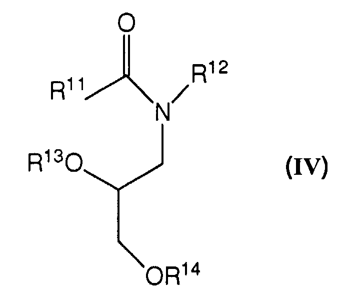 wherein R¹¹ is a linear or branched, saturated, or unsaturated, aliphatic hydrocarbon group having from 1 to 10 carbon atoms which may contain a hydroxy group, ester group and/or an ether group; R ¹² is a linear or branched, saturated or unsaturated aliphatic hydrocarbon group having from 7 to 48 carbon atoms; R ¹³ and R ¹⁴ are the same or different and each is selected from the group consisting of hydrogen, a sulfate group, a phosphate group, or a sugar group.

The neoceramide can be prepared according to the procedure described by Cho et al., U.S. Patent No. 5,476,671.

Specific preferred examples of ceramides, pseudoceramides and neoceramides are represented by the following Formulae below:

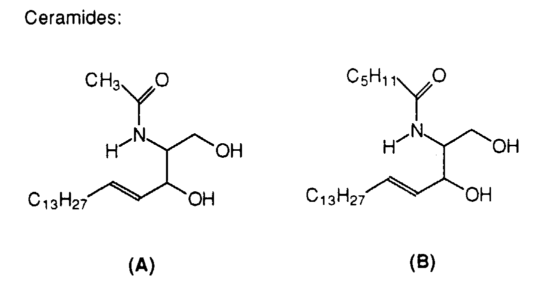

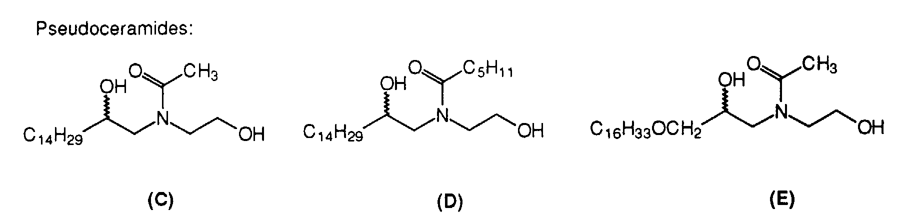

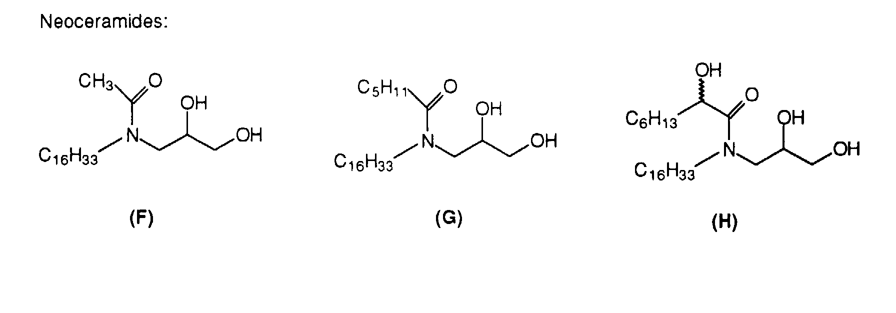

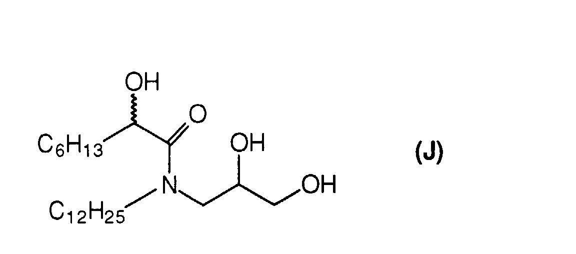

The amount of the short-chain lipid material in the composition preferably is in the range of from 0.0001% to 50% by weight of the composition, more preferably from 0.001% to 50%. For both embodiments of the present compositions, it is even more preferably from 0.001% to 10%, most preferably from 0.01% to 1%.

### Cosmetically Acceptable Vehicle:

The composition according to the invention also comprises a cosmetically acceptable vehicle to act as a dilutant, dispersant or carrier for the active components in the composition, so as to facilitate their distribution when the composition is applied to the skin, hair and/or nails.

Vehicles other than or in addition to water can include liquid or solid emollients, solvents, humectants, thickeners and powders. An especially preferred nonaqueous carrier is a polydimethyl siloxane and/or a polydimethyl phenyl siloxane. Silicones of this invention may be those with viscosities ranging anywhere from about 10 to 10,000,000 mm^{2/}s (centistokes) at 25°C. Especially desirable are mixtures of low and high viscosity silicones. These silicones are available from the General Electric Company under trademarks Vicasil, SE and SF and from the Dow Corning Company under the 200 and 550 Series. Amounts of silicone which can be utilized in the compositions of this invention range anywhere from 5% to 95%, preferably from 25% to 90% by weight of the composition.

The cosmetically acceptable vehicle will usually form from 5% to 99.9%, preferably from 25% to 80% by weight of the composition, and can, in the absence of other cosmetic adjuncts, form the balance of the composition.

### Optional Skin Benefit Materials and Cosmetic Adjuncts:

An oil or oily material may be present, together with an emulsifier to provide either a water-in-oil emulsion or an oil-in-water emulsion, depending largely on the average hydrophilic-lipophilic balance (HLB) of the emulsifier employed.

In a preferred embodiment of the invention, the inventive compositions further include hydroxy acid and/or keto acid which are particularly effective in combination with a flavanone.

Hydroxyacids - enhance proliferation and increase ceramide biosynthesis in keratinocytes, increase epidermal thickness, and increase desquamation of normal skin resulting in smoother, younger looking skin.

The hydroxy acid can be chosen from α-hydroxy acids, β-hydroxyacids, other hydroxycarboxylic acids (e.g., dihydroxycarboxylic acid, hydroxy-dicarboxylic, hydroxytricarboxylic) and mixtures thereof or combination of their stereoisomers (DL, D or L).

Preferably the hydroxy acid is chosen from α-hydroxy acids having the general structure (13): 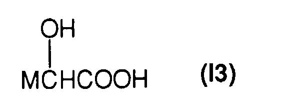 where M is H - or CH ₃ C_{f}H_{g})ₕ - , in which h is 0 or 1, f is an integer of from 1 to 27, and g is an integer of from 2 to 54.

Even more preferably, the hydroxy acid is chosen from 2-hydroxyoctanoic acid, hydroxylauric acid, lactic acid, and glycolic acid, and mixtures thereof. When stereo isomers exist, L-isomer is most preferred.

The keto acids can be chosen from α-keto acids, β-keto acids and mixtures thereof.

A particularly preferred α-keto acid is 2-keto octanoic acid.

Preferably the amount of the hydroxy acid component present in the composition according to the invention is from 0.01% to 20%, more preferably from 0.05% to 10% and most preferably from 0.1% to 3% by weight.

Emollients are often incorporated into cosmetic compositions of the present invention. Levels of such emollients preferably range from 0.5% to 50%, more preferably between 5% and 30% by weight of the total composition.

Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1% to 20% by weight, preferably from 0.5% to 10% by weight of the composition. Exemplary thickeners are cross-linked polyacrylate materials available under the trademark Carbopol® from the B.F. Goodrich Company. Gums may be employed such as xanthan, carrageenan, gelatin, karaya, pectin and locust beans gum. Under certain circumstances the thickening function may be accomplished by a material also serving as a silicone or emollient. For instance, silicone gums with viscosity in excess of 10mm^{2/}s (centistokes) and esters such as glycerol stearate have dual functionality.

Various other types of active ingredients may be present in cosmetic compositions of the present invention.

Actives are defined as skin or hair benefit agents other than emollients and other than ingredients that merely improve the physical characteristics of the composition. Although not limited to this category, general examples include sunscreens, tanning agents, skin anti-wrinkling agents, anti-dandruff agents, and hair growth stimulants.

Many cosmetic compositions, especially those containing water, must be protected against the growth of potentially harmful microorganisms. Preservatives are, therefore often necessary. Suitable preservatives include alkyl esters of p-hydroxybenzoic acid, hydantoin derivatives, propionate salts, and a variety of quaternary ammonium compounds.

Powders may be incorporated into the cosmetic composition of the invention. These powders include chalk, talc, Fullers earth, kaolin, starch, smectites clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the cosmetic compositions. These ingredients may include coloring agents, opacifiers and perfumes. Amounts of these other adjunct minor components may e.g. range anywhere from 0.001% up to 20% by weight of the composition.

### Use of the Composition:

The composition according to the invention is intended primarily as a cosmetic product for topical application to human skin, especially as an agent for reducing the permeability to water of the skin, particularly when the skin is dry or damaged, in order to reduce moisture loss and generally to enhance the quality and flexibility of skin. The composition can also be applied to hair and nails.

In use, a small quantity of the composition, for example from 1 to 5ml, is applied to exposed areas of the skin, from a suitable container or applicator and, if necessary, it is then spread over and/or rubbed into the skin using the hand or fingers or a suitable device.

### Product Form and Packaging:

The topical skin and/or hair treatment composition of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas or above. The composition can be packaged in a suitable container to suit its viscosity and intended use by the consumer. For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar. The invention accordingly also provides a closed container containing a cosmetically acceptable composition as herein defined.

The composition may also be included in capsules such as those described in U.S. Patent 5,063,507.

The following specific examples further illustrate the invention.

### EXAMPLES

### EXAMPLES 1 AND 2: TG-1 ASSAY

### Materials:

Naringin, naringenin, hesperedin, eriodictyol, taxifolin, apigenin, fisetin, daidzein, quercetin, formononetin, chrysin were obtained from Indofine Chemical Company, Inc., Somerville, New Jersey, USA. Hesperetin, neohesperedin and epicatechin were obtained from Sigma Chemical Company.

### Methods:

### Cell Culture:

Human Epidermal Keratinocytes (Clonetics) were grown on a feeding layer of 3T3 MTM fibroblasts in Complete Media (DME + f-12, 1M Hepes, Adenine, Hydrocortisone, Cholera Toxin, Insulin, EGF, FBS, and Penn-Strep) and in the presence of 0.09 mM Calcium.

Following trypsin treatment, the keratinocytes were seeded at 3 x 10 ³ cells/well in 96-well tissue culture treated plates (Costar) in 200µl of KSFM (Life Technologies) + L-glutamine, 0.09mM Calcium, BPE, and EGF. Plates were seeded using the middle 60 wells for the experiment and the bottom ten wells for the secondary antibody control.

### Dosing:

After 96 hours incubation, the medium was removed, and fresh medium was added along with a test compound, at concentration specified in the Tables below, within complete KSFM. High Calcium (1.2mM) acted as the positive control.

Plates were pulled, washed, and stored at -70°C, after 24 hours, 48 hours, 72 hours, and 96 hours of incubation with the test compound.

### Transglutaminase Assay:

The 96-well plates prepared above were freeze/thawed (-70°C/room temp.) twice. Aliquots of 200µl of ice cold 1:1 EtOH/acetone were added to each well, then incubated at 4°C for 30 minutes. The ethanol/acetone was aspirated off, then air dried in a fume hood for 30 minutes.

The wells were blocked with 200µl/well of 5% Milk in BSA, then aspirated and treated with 100µl/well of anti-human transglutaminase primary mouse monoclonal antibody for two hours at room temperature. The plates were washed six times with 100µl/well of 1% BSA in PBS + 0.1% Tween using a Dynatech mechanical washer.

The wells were then treated with 100µl/well Anti-mouse Ig, peroxidase-linked species-specific F(ab') ₂ fragment from sheep (Amersham) and incubated at room temperature for two hours. The plates were washed six times with 100µl/well of 1%BSA in PBS + 0.05% Tween using the Dynatech mechanical washer.

Finally, the wells were treated with 100µl of freshly prepared citrate buffer (0.1M Citric Acid + 0.2M Sodium phosphate dibasic) in the presence of O-phenylenediamine and hydrogen peroxide. After five minutes in the dark, 50µl/well of 4N H₂SO₄ was added to stop the reaction. The plates were immediately measured for absorbance at OD 490nm in an ELISA plate reader (Dynatech).

### EXAMPLE 1

A series of flavanones, iso-flavones, flavones, flavanone glycosides and epicatechin were subjected to the transglutaminase (TG-1) assay at 5 and 15 µg/ml. The results that were obtained (after 72 hour incubation) are summarized in Table 1 (as increase in TG-1 or no effect on TG-1).

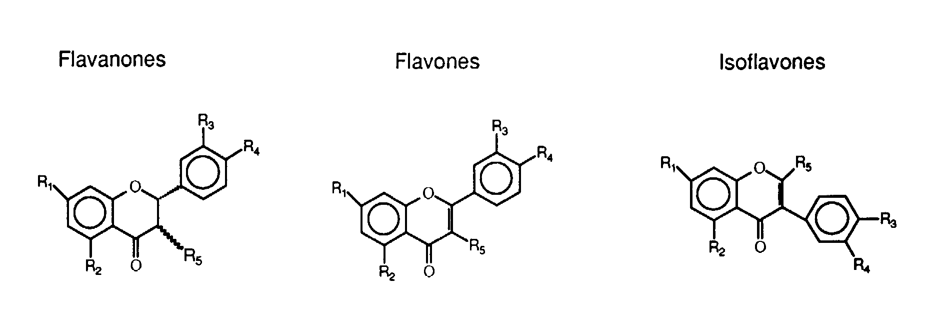

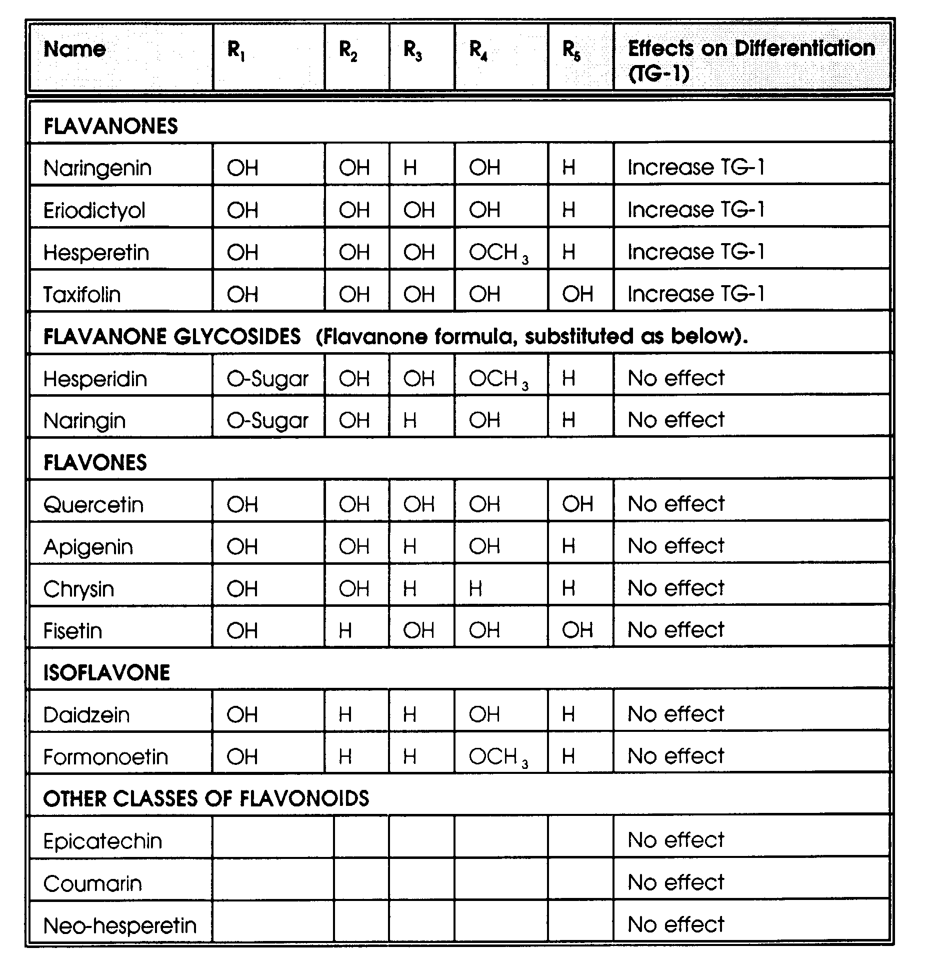

As demonstrated by the results in Table 1 above, the flavanones (i.e., naringenin, hesperetin, eriodictyol, and taxifolin) stimulated TG-1 production, indicating prodifferentiating activity. This trend was very specific to flavanones and not observed with other classes of flavonoids listed in Table 1.

We observed that apigenin and quercetin disrupted the cell membranes leaving a layer of debris around the cells and caused what looked like a toxic effect upon the morphology of the cells. Most of flavones appear to have a cytotoxic effect on keratinocytes at these concentrations. The same effects were observed with fisetin and formononetin. Daidzein and epi-catechin showed no effects in this time course study. Neither stimulation nor inhibition of differentiation processes were observed. The cells showed no morphological changes compared to those of the control.

### EXAMPLE 2

As demonstrated in Example 1, flavanones increased TG-1 production. Table 2 summarizes the results that were obtained for the following flavanones: hesperetin, naringenin, eriodictyol and taxifolin. The TG-1 production was measured at various times against the control (solution without the active).

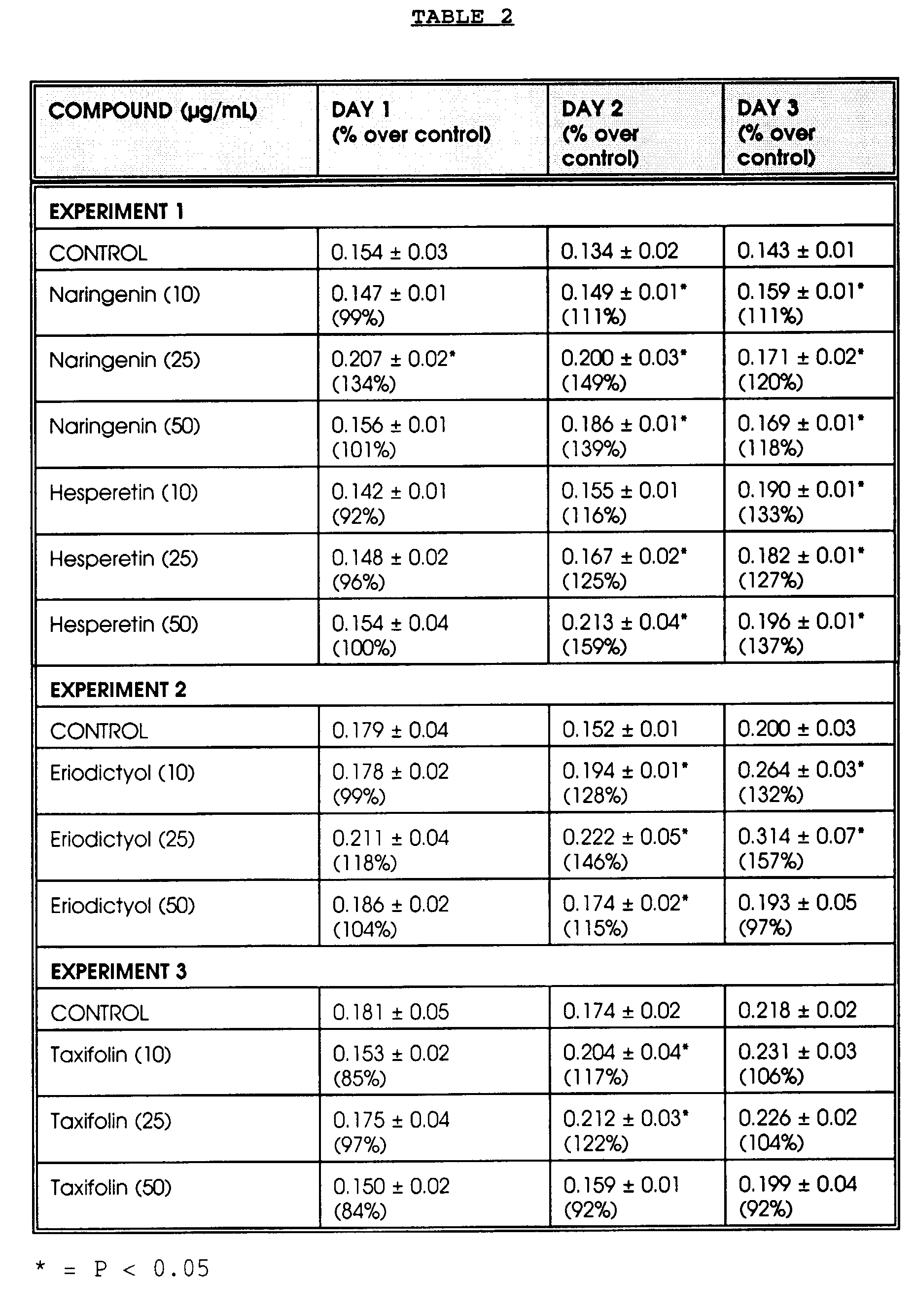

Hesperetin, naringenin, taxifolin and eriodictyol all stimulated differentiation to the same extent or higher than the control. Incubation with flavanones for 2 or 3 days almost universally resulted in significantly higher TG-1 production than incubation with a control.

### EXAMPLE 3

### Immunohistochemistry of Keratinocytes for Transglutaminase

### Method:

Keratinocytes were plated in 4-well chamber slides (Nunc) in serum-free keratinocyte growth medium containing 0.09 mM Ca. After four days in culture, some of the wells were switched to 1.2 mM Ca medium to induce differentiation. Other wells were treated with 10, 25 or 50 µg/ml of hesperetin or naringenin. After 48 hours of treatment, all the wells were washed with PBS, fixed with ice cold ethanol:acetone (1:1) and immunostained for transglutaminase-1 using the commercially available antibody BC1 as follows: After rinsing two times with PBS, the wells were incubated in PBS buffered saline (TBS pH 8.0) containing 5% nonfat milk. The wells were then incubated with the first antibody (1 to 2000 dilution) in the above buffer for two hours at room temperature. Wells were washed three times with the TBS containing 1% BSA and 0.05% Tween-20 before incubation with the second antibody (1-3000 dilution of goat antimouse antibody conjugated to horseradish peroxidase) overnight at 4°C. Color development was achieved using dimethylformamide and counterstained lightly using hematoxylin to visualize the nuclei. Slides were then mounted in aquamount and viewed under microscope.

### Results:

Immunohistochemistry for TG-1 showed that the keratinocytes in the low calcium medium grew as a monolayer of cuboidal cells with a well formed nucleus. Only very few cells stained for with the antibody suggesting the minimal level of expression of TG-1 under these low calcium conditions. Keratinocytes switched to 1.2 mM Ca for 48 hours formed multilayered stratified colonies with tightly packed cells. These colonies, especially the middle of the colonies, showed strong reactivity to TG-1 antibody suggesting the differentiated nature of these cultures. The differentiation states of keratinocytes treated with the different flavonoids in low calcium medium were similar to the high calcium controls judging both by morphology and by TG-1 immunostaining. A concentration dependence was also evident on a microscopic picture: low concentrations (10 µg/ml) showed lower TG-1 levels than the higher concentrations (50 µg/ml).

### EXAMPLE 4

TG-1 assay as described in Example 1 was conducted for various combinations of flavanones. The results that were obtained are summarized in Table 3.

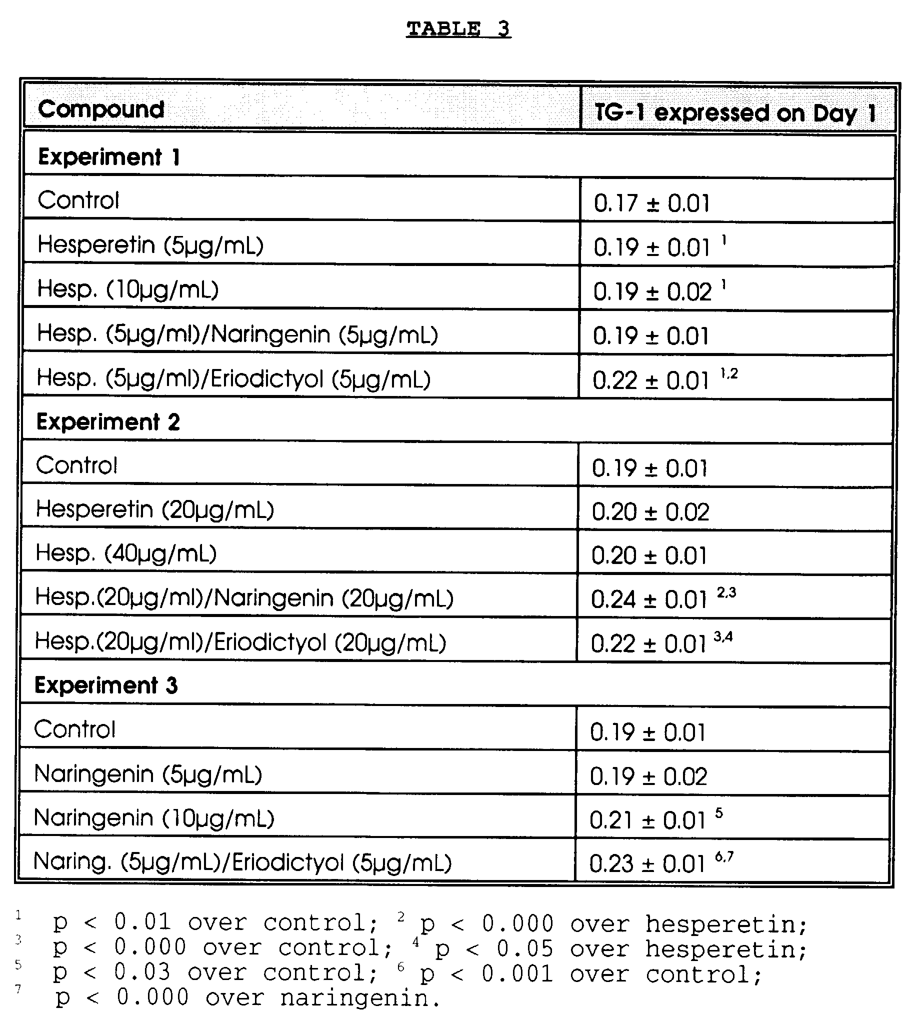

The results in Table 3 above demonstrate that a combination of two flavanones increased TG-1 production over a control, even at a low concentration and on Day 1.

Example 4 demonstrates that a combination of two flavanones, particularly hesperetin or naringenin with eriodictyol results in a greater increase in TG-1 production than that attained by naringenin or eriodictyol alone.

### EXAMPLE 5

TG-1 assay as described in Example 1 was conducted for combinations of flavanones with short-chain lipids. The results that were obtained are summarized in Table 4.

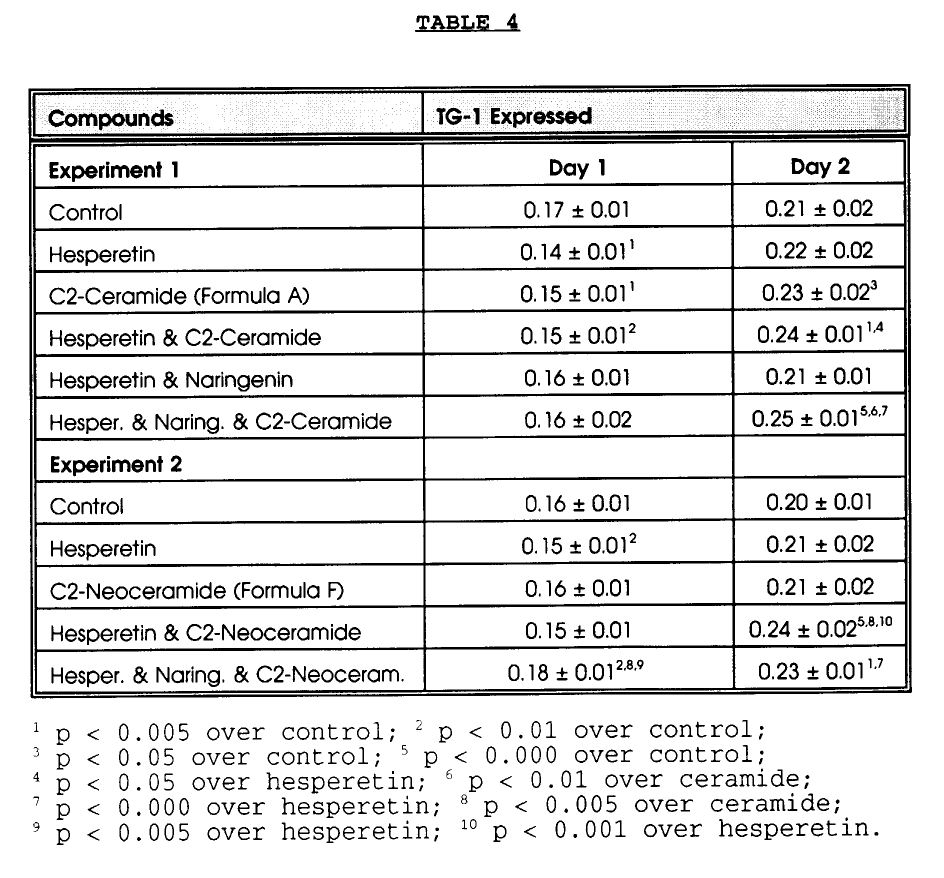

Examples 6 - 11 illustrate topical compositions according to the present invention. The compositions can be processed in conventional manner. They are suitable for cosmetic use. In particular the compositions are suitable for application to wrinkled, flaky, aged and/or photodamaged skin, to improve the appearance and feel thereof as well as for application to healthy skin to prevent or retard deterioration thereof.

### EXAMPLE 6

This example illustrates a high internal phase water-in-oil emulsion.

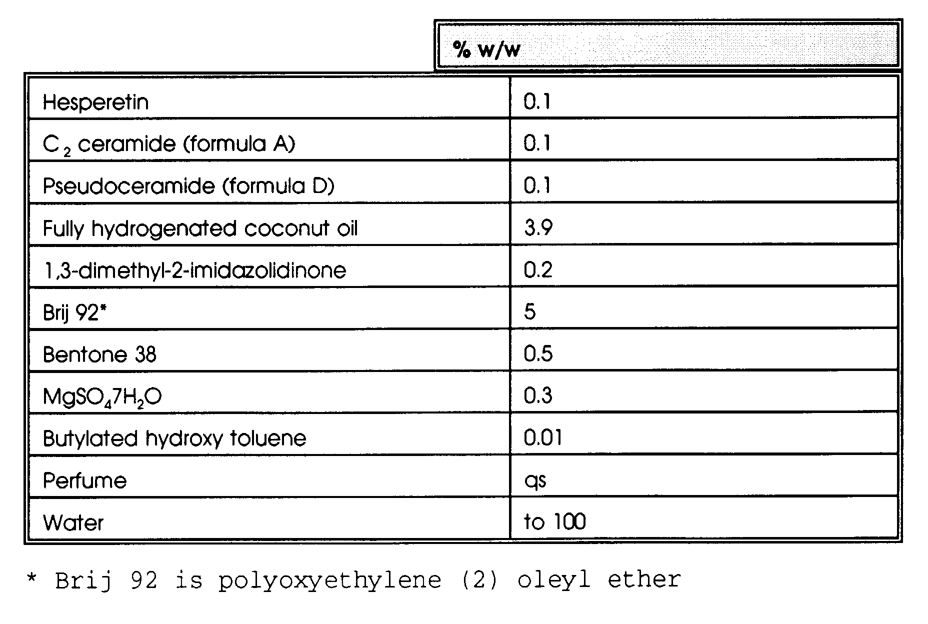

### EXAMPLE 7

This example illustrates an oil-in-water cream.

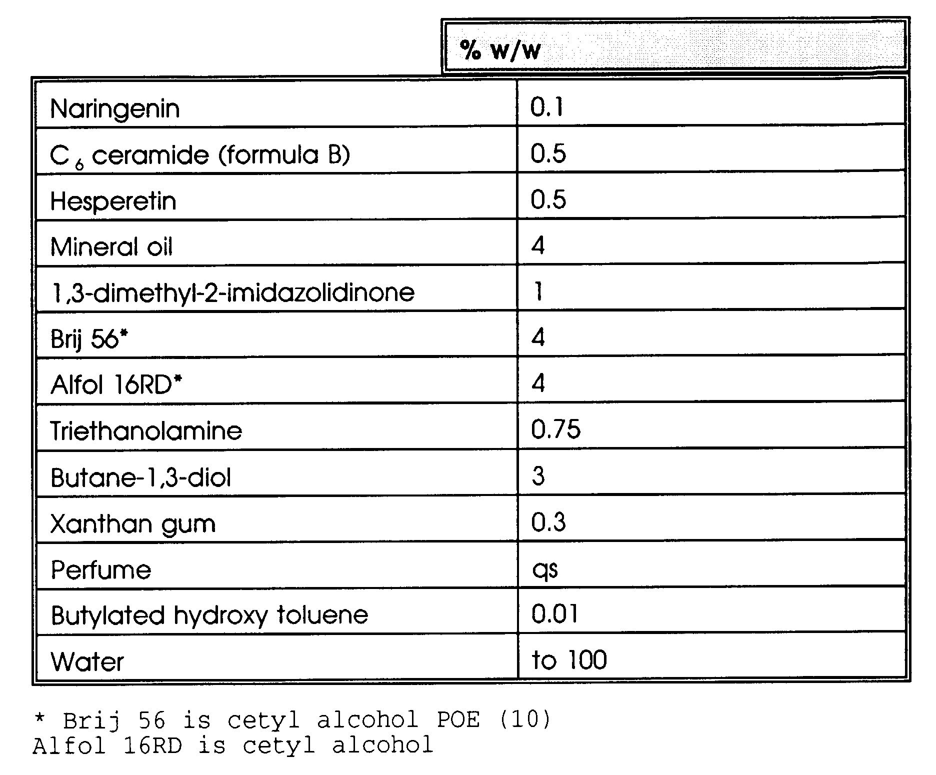

### EXAMPLE 8

This example illustrates an alcoholic lotion.

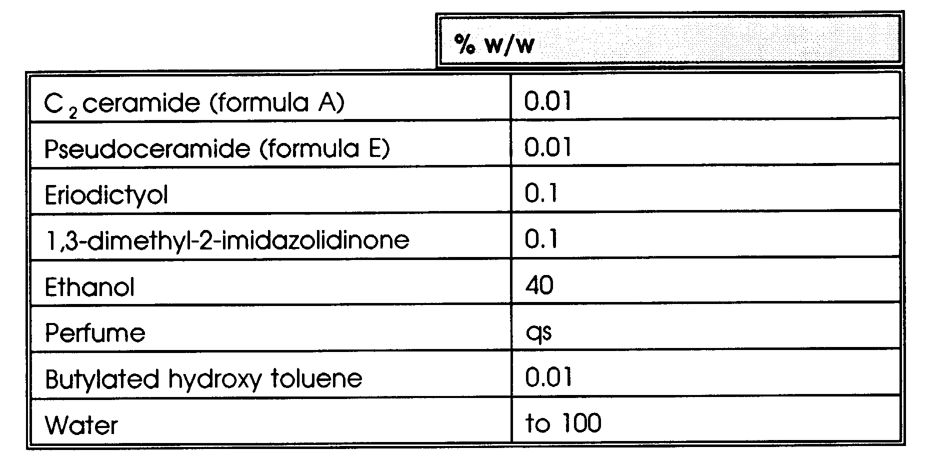

### EXAMPLE 9

This example illustrates another alcoholic lotion.

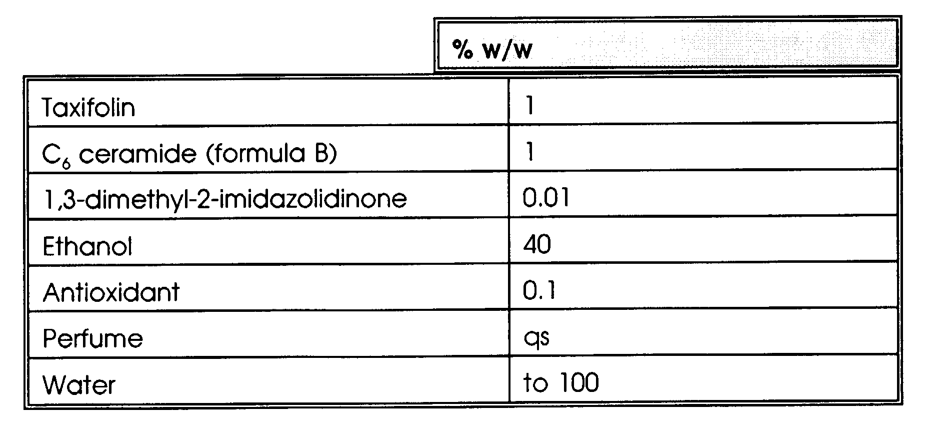

### EXAMPLE 10

This example illustrates a suncare cream.

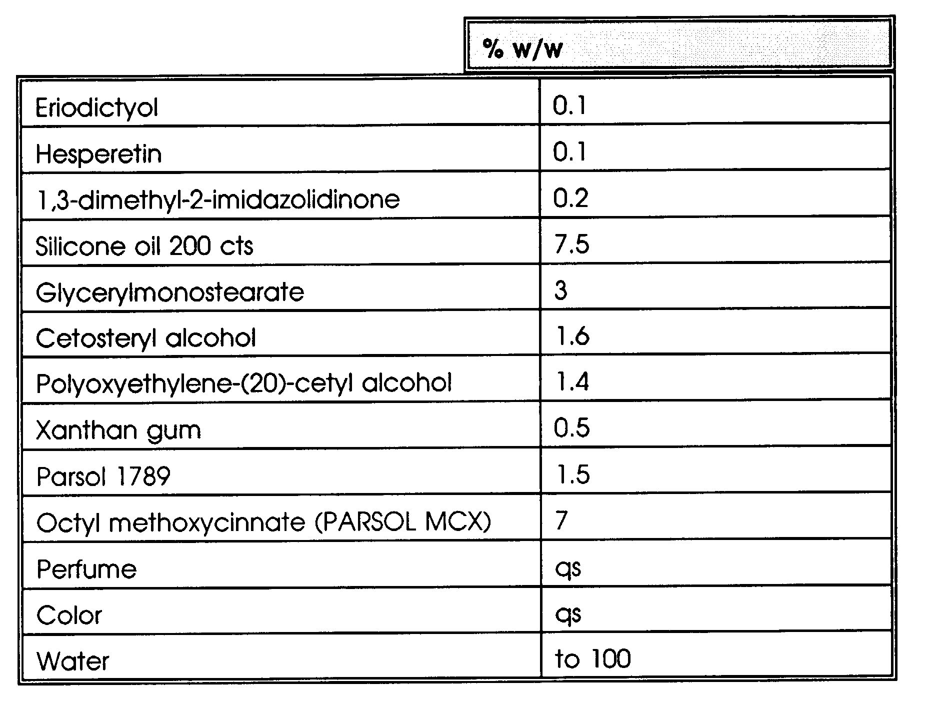

### EXAMPLE 11

This example illustrates a non-aqueous skin care composition.

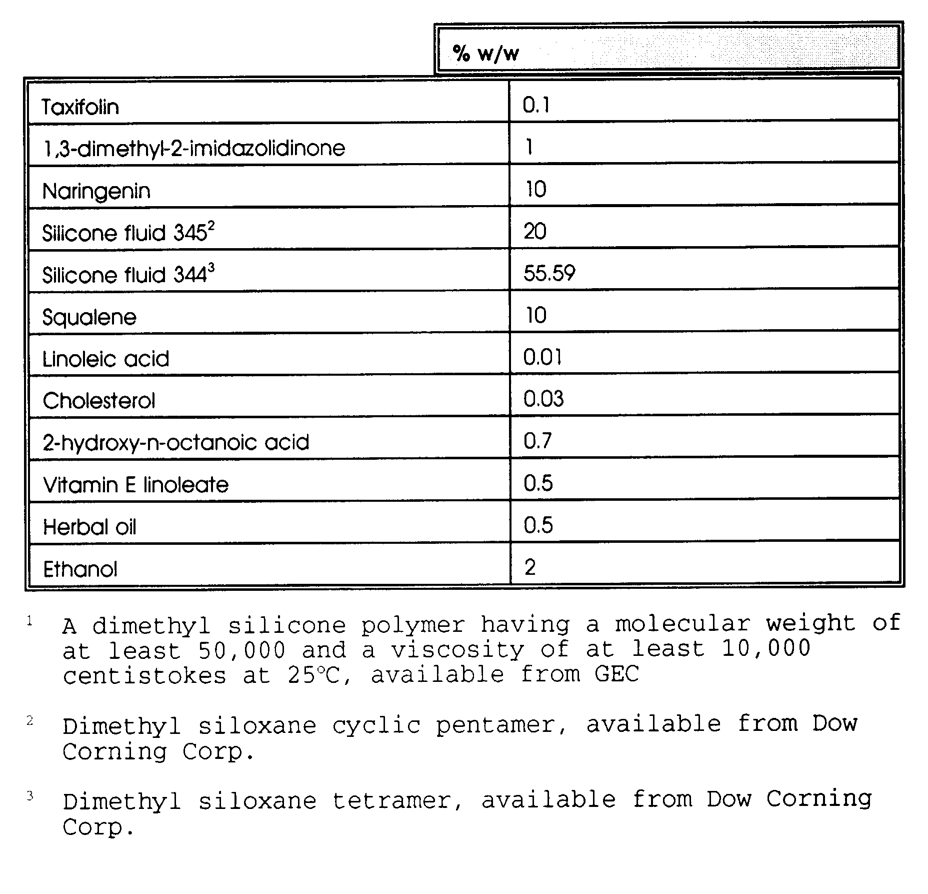

## Claims

1. A topical composition comprising:
(a) from 0.001% to 5.0% by weight of a first flavanone selected from the group consisting of eriodictyol, taxifolin, and mixtures thereof;
(b) from 0.001% to 5.0% by weight of a second flavanone selected from the group consisting of hesperetin, naringenin, and mixtures thereof; and
(c) a cosmetically acceptable vehicle for the flavanones.

2. The composition of claim 1 wherein the weight ratio of the second flavanone to the first flavanone is in the range of from 1:5 to 5:1.

3. The composition according to claim 1 or claim 2, further comprising from 0.001 to 50 wt. % of a ceramide selected from the group consisting of a short-chain ceramide, pseudoceramide and neoceramide.

4. A topical composition comprising:
(a) from 0.001 to 5.0 wt. % of a flavanone selected from the group consisting of eriodictyol, taxifolin, hesperetin, naringenin, and mixtures thereof;
(b) from 0.001 to 50 wt. % of a ceramide selected from the group consisting of a short-chain ceramide, pseudoceramide, and neoceramide; and
(c) a cosmetically acceptable vehicle for the flavanone and the ceramide.

5. The composition of claim 4 wherein the weight ratio of the flavanone to the ceramide is in the range of from 1:5 to 5:1.

6. A cosmetic method of enhancing keratinocyte differentiation in skin, the method comprising applying to the skin a composition according to any one of claims 1-5.

7. A cosmetic method for treating the appearance of wrinkled, flaky, aged, or photodamaged skin comprising applying to the skin a composition according to any one of claims 1-5.
